# EUROPÄISCHE PATENTSCHRIFT

(11) **EP 1 838 171 B1**
(45) Veröffentlichungstag und Bekanntmachung des Hinweises auf die Patenterteilung: **18.07.2012**
(21) Anmeldenummer: 06706424.6
(22) Anmeldetag: 26.01.2006
(51) Int. Cl.: A61K 8/99, A61Q 19/00, C11B 5/00, A61K 36/02, A23L 1/30, A23K 1/16, A61K 8/97, A23K 1/00

(54) **HERSTELLUNG UND ANWENDUNG EINES ANTIOXIDATIV WIRKSAMEN EXTRAKTES AUS CRYPTHECODINIUM SP**
PRODUCTION AND USE OF AN ANTIOXIDANT EXTRACT FROM CRYPTHECODINIUM SP
FABRICATION ET UTILISATION D'UN EXTRAIT DE CRYPTHECODINIUM SP A EFFET ANTIOXYDANT

(30) Priorität: 26.01.2005 DE 102005003624
(43) Veröffentlichungstag der Anmeldung: 03.10.2007
(73) Patentinhaber: Lonza Ltd, 4002 Basel (CH)
(72) Erfinder: FABRITIUS, Dirk, 91522 Ansbach (DE); HOHMANN, Doreen, 65795 Hattersheim (DE)
(74) Vertreter: Mai, Dörr, Besier
(86) Internationale Anmeldenummer: PCT/EP2006/000676
(87) Internationale Veröffentlichungsnummer: WO 2006/079533

(56) Entgegenhaltungen:
- EP-A- 1 178 118
- EP-A- 1 359 224
- WO-A-00/54575
- WO-A-97/37032
- WO-A-03/033631
- WO-A-03/056939
- WO-A-03/092628
- US-A1- 2004 106 584
- FRANKEL EDWIN N; SATUE-GRACIA TERESA; MEYER ANNE S; GERMAN J BRUCE: "Oxidative stability of fish and algae oils containing long-chain polyunsaturated fatty acids in bulk and in oil-in-water emulsions" J.AGRIC.FOOD CHEM., Bd. 50, 2002, Seiten 2094-2099, XP002387550
- BECKER C C ET AL: "DEVELOPING FUNCTIONAL FOODS CONTAINING ALGAL DECOSAHEXAENOIC ACID" FOOD TECHNOLOGY, INSTITUTE OF FOOD TECHNOLOGISTS, CHICAGO, IL, US, Bd. 52, Nr. 7, Juli 1998 (1998-07), Seiten 68-71, XP000771910 ISSN: 0015-6639
- FISCH K M; BOHM V; WRIGHT A D; KONIG G M: "Antioxidative meroterpenoids from the brown alga Cystoseira crinita" JOURNAL OF NATURAL PRODUCTS, Bd. 66, 2003, Seiten 968-975, XP002387551
- BOHM VOLKER; PUSPITASARI-NIENABER NI LUH; FERRUZZI MARIO G; SCHWARTZ STEVEN J: "Trolox equivalent antioxidant capacity of different geometrical isomers of alpha-carotene, beta-carotene, lycopene, and zeaxanthin" JOURNAL OF AGRICULTURAL AND FOOD CHEMISTRY, Bd. 50, 2002, Seiten 221-226, XP002387552
- PELLEGRINI NICOLETTA; SERAFINI MAURO; COLOMBI BARBARA; DEL RIO DANIELE; SALVATORE SARA; BIANCHI MARTA; BRIGHENTI FURIO: "Total antioxidant capacity of plant foods, beverages and oils consumed in Italy assessed by three different in vitro assays." JOURNAL OF NUTRITION, Bd. 133, 2003, Seiten 2812-2819, XP002387553
- BUSHMAN B SHAUN; PHILLIPS BLISS; ISBELL TERRY; OU BOXIN; CRANE JIMMIE M; KNAPP STEVEN J: "Chemical composition of caneberry (Rubus spp.) seeds and oils and their antioxidant potential" JOURNAL OF AGRICULTURAL AND FOOD CHEMISTRY, Bd. 52, 2004, Seiten 7982-7987, XP002387554
- LIEBERT MARIA; LICHT URTE; BOEHM VOLKER; BITSCH ROLAND: "Antioxidant properties and total phenolics content of green and black tea under different brewing conditions" ZEITSCHRIFT FUER LEBENSMITTEL-UNTERSUCHUNG UND -FORSCHUNG A, Bd. 208, 1999, Seiten 217-220, XP002387555
- HOFFMAN ET AL.: "Maturation of visual acuity is accelerated in breast-fed term infants fed baby food containing DHA-enriched egg yolk" JOURNAL OF NUTRITION, Bd. 134, 2004, Seiten 2307-2313, XP002387556

## Beschreibung

Die vorliegende Erfindung betrifft einen Extrakt aus *Crypthecodinium sp*., ein Verfahren zu seiner Herstellung sowie seine Verwendung, insbesondere zur antioxidativen Stabilisierung von Fettsäure-Zusammensetzungen, welche eine oder mehrere hochungesättigte, langkettige Fettsäuren und/oder eine oder mehrere hochungesättigte, langkettige Fettsäureester enthalten.

Hochungesättigte, langkettige Fettsäuren (Polyunsaturated Fatty Acids; PUFAs) stellen essentielle Fettsäuren des menschlichen Stoffwechsels dar. PUFAs können in zwei große Gruppen unterteilt werden. Neben der Gruppe der ω-6 PUFAs, die ausgehend von Linolsäure formuliert werden, gibt es die Gruppe der ω-3 PUFAs, die ausgehend von der α-Linolensäure aufgebaut werden.

PUFAs sind wichtige Bausteine der Zellmembranen, der Retina und der Hirnhaut und Vorläufer für wichtige Hormone, beispielsweise für die Prostaglandine, die Thromboxane und die Leukotriene.

Neben der Funktion als Baustein hat sich im Laufe der letzten Jahre immer mehr gezeigt, dass PUFAs direkt vielfältige positive Wirkungen auf den menschlichen Organismus bzw. Erkrankungen haben.

Eine Vielzahl von klinischen Studien haben gezeigt, dass PUFAs z.B. bei Krebs, rheumatischer Arthritis, Bluthochdruck und Neurodermitis und vielen anderen Erkrankungen einen wichtigen Beitrag zur Heilung oder Linderung leisten können. Dabei ist die Verwendung von Docosahexaensäure (DHA; all-cis 4,7,10,13,16,19 Docosahexaensäure) und ihren Derivaten, insbesondere von DHA-Estem oftmals besonders vorteilhaft, weil sie (insbesondere die Ethylester und die Triglyceride) dazu neigen, einen angenehmen Geschmack zu haben und leicht durch das Verdauungssystem absorbiert zu werden. Diese Erkenntnisse waren ursächlich dafür verantwortlich, dass internationale Institutionen und Behörden Empfehlungen ausgesprochen haben, die die tägliche Aufhahmemenge von PUFAs regeln.

PUFAs können vom Menschen nicht *de-novo* synthetisiert werden, da ihnen die Enzymsysteme fehlen, die eine Doppelbindung in die Kohlenstoffkette an Positionen > C9 einführen können (fehlende Δ12-Desaturase). Erst durch die Zufuhr von sogenannten Vorläufer-Fettsäuren (Precursoren, z.B. α-Linolensäure) über die Nahrung ist der Mensch in der Lage, hochungesättigte Fettsäuren zu synthetisieren. Ob diese Menge jedoch ausreicht, um den Bedarf an hochungesättigten Fettsäuren zu decken, ist umstritten.

Der allergrößte Teil der essentiellen Fettsäuren wird durch die Nahrung aufgenommen. Insbesondere Pflanzenöle sind mit ω-6 Fettsäuren, (beispielsweise enthält Nachtkerzenöl γ-Linolensäure (GLA)), jedoch nur bis zu einer Kettenlänge von C18, und Fischöle bzw. Öle aus Mikroorganismen mit ω-3 Fettsäuren (z.B. enthält Lachsöl Eicosapentaensäure (EPA) und Docosahexaensäure (DHA; all-cis 4,7,10,13,16,19 Docosahexaensäure)) angereichert. Grundsätzlich stellen Fischöle und Öle aus Mikroorganismen die einzige kommerzielle Quelle für hochungesättigte Fettsäuren dar. Im Allgemeinen ist jedoch der Gehalt an den gewünschten PUFAs zu gering und diese liegen in einer Mischung vor, wobei antagonistisch wirkende PUFAs ebenfalls enthalten sein können. Um die empfohlene tägliche Dosis an PUFAs zu sich zu nehmen, muss also eine hohe Ölmenge aufgenommen werden. Insbesondere trifft das auf solche Patienten zu, die hohe Dosen von PUFAs zu sich nehmen müssen (beispielsweise bei Cystischer Fibrose). Um eine möglichst gezielte Wirkung der einzelnen PUFAs zu erreichen, müssen angereicherte bzw. hochreine PUFAs eingesetzt werden. Es besteht daher im Stand der Technik ein hoher Bedarf an hochreinen PUFAs.

Zahlreiche Verfahren wurden einzeln oder in Kombination verwendet, um bestimmte Fettsäuren und ihre Derivate aus einer Vielzahl natürlich vorkommender Quellen zu isolieren (oder zumindest aufzukonzentrieren) und zurückzugewinnen. Diese Verfahren schließen die fraktionierte Kristallisation bei niedrigen Temperaturen, die molekulare Destillation, die Harnstoffadukt-Kristallisation, die Extraktion mit Metallsalzlösungen, die superkritische Fluidfraktionierung auf gegenläufigen Kolonnen und HPLC-Verfahren ein.

Aufgrund ihrer Oxidationsempfindlichkeit müssen PUFAs in der Regel durch Zugabe von geeigneten Antioxidantien stabilisiert werden. Kommerziell werden für diesen Zweck vor allem natürliche Tocopherole, insbesondere aus Sojaöl extrahierte Gemische von α-, β-, γ-, δ-Tocopherol, und/oder Tocotrienole eingesetzt. Des Weiteren ist bekannt, dass manche Verbindungen, wie beispielsweise Ascorbylpalmitat, synergistisch wirken können. Sie werden daher zusätzlich zum Tocopherol eingesetzt.

Die Wirkung der natürlichen Antioxidantien steigt jedoch nicht unbegrenzt mit steigender Konzentration an. Beispielsweise kehrt sich bei α-Tocopherol die Aktivität schon bei 100 ppm um und es tritt eine prooxidative Wirkung auf. Dies bedeutet, dass eine Überdosierung sich auch negativ auswirken kann.

Alternativ schlägt die Druckschrift WO03092628 die Verwendung eines schonend aufgearbeiteten Öls vor. Die Aufbereitung soll dabei derart erfolgen, dass man eine eine mehrfach ungesättigte Fettsäure-enthaltende Biomasse zunächst mit einem Enzym umgesetzt und das Lipid anschließend isoliert. Obwohl das auf diese Weise erhältliche Öl zunächst scheinbar nicht so stark oxidiert wird, weist es dennoch die für mehrfach ungesättigte Fettsäuren charakterische Oxidationsempfindlichkeit auf.

WO 00 54575 beschreibt Fettsäure- Zusammensetzungen, die durch Zügabe von Phospholipiden stabilisiert werden.

In Anbetracht dieses Standes der Technik war es daher Aufgabe der vorliegenden Erfindung, Möglichkeiten zur verbesserten antioxidativen Stabilisierung von Fettsäure-Zusammensetzungen aufzuzeigen. Die Steigerung der antioxidativen Wirkung sollte dabei möglichst ohne Zusatz von gesundheitsgefährdenden Substanzen erreicht werden, um Anwendungen der Fettsäure-Zusammensetzung im Lebensmittelbereich ohne Bedenken zu ermöglichen.

Eine weitere Aufgabe der vorliegenden Erfindung bestand in der Angabe eines Verfahrens zur Herstellung der erfindungsgemäßen Fettsäure-Zusammensetzung, welches ihre Herstellung auf möglichst einfache Art und Weise, großtechnisch und kostengünstig erlaubt.

Darüber hinaus sollten besonders vorteilhafte Anwendungsgebiete der erfindungsgemäßen Fettsäure-Zusammensetzung aufgezeigt werden. Gelöst werden diese sowie weitere Aufgaben, die zwar nicht wörtlich genannt werden, sich aber aus den hierin diskutierten Zusammenhängen wie selbstverständlich ableiten lassen oder sich aus diesen zwangsläufig ergeben, durch einen antioxidativ wirksamen Extrakt aus *Crypthecodinium sp..* Zweckmäßige Abwandlungen des erfiridungsgemäßen Extraktes werden in den auf Anspruch 1 rückbezogenen Unteransprüchen beschrieben. Die Ansprüche 11 bis 19 stellen antioxidativ stabilisierte Fettsäure-Zusammensetzungen unter Schutz. Der Verfahrensanspruch schützt eine besonders geeignete Herstellungsweise der erfindungsgemäßen Fettsäure-Zusammensetzung und die Verwendungsansprüche beschreiben besonders vorteilhafte Anwendungsgebiete der erfindungsgemäßen Fettsäure-Zusammensetzung.

Dadurch, dass man einen antioxidativ wirksamen Extrakt aus *Crypthecodinium sp*. zur Verfügung stellt, gelingt es auf nicht ohne weiteres vorhersehbare Weise, einen Extrakt mit besonders hoher antoxidativer Wirkung zugänglich zu machen, welcher sich insbesondere für die antioxidative Stabilisierung von Fettsäure-Zusammensetzungen, vor allem von solchen Fettsäure-Zusammensetzungen, die mindestens eine ungesättigte Fettsäure und/oder mindestens einen ungesättigten Fettsäureester enthalten, eignet. Dabei wird die Steigerung der antioxidativen Wirkung erfindungsgemäß ohne Zusatz von gesundheitsgefährdenden Substanzen erreicht, d. h. ein Einsatz der erfindungsgemäßen Fettsäure-Zusammensetzung im Lebensmittelbereich ist ohne Bedenken möglich. So wird *Crypthecodinium cohnii*-Öl bereits in der Säuglingsernährung eingesetzt und ist in den USA als GRAS eingestuft ("Generally regarded as safe").

Die erfindungsgemäße Fettsäure-Zusammensetzung kann auf einfache Art und Weise, großtechnisch und kostengünstig hergestellt werden.

Die Fettsäure-Zusammensetzung enthält gemäß der vorliegenden Erfindung mindestens einen antioxidativ wirksamen Extrakt aus *Crypthecodinium sp.,* vorzugsweise aus *Crypthecodinium cohnii.* Der Begriff "Fettsäure-Zusammensetzung" umfasst in diesem Zusammenhang sowohl Zusammensetzungen, die freie Fettsäuren enthalten, als auch Zusammensetzungen, die Fettsäurederivate, vorzugsweise Fettsäureester, insbesondere Fettsäuretriglyceride, wobei die Fettsäurereste prinzipiell gleich oder verschieden sein können.

Fettsäuren bezeichnen erfindungsgemäß aliphatische Carbonsäuren, die gesättigt oder ein- oder mehrfach ungesättigt sein können und vorzugsweise 6 bis 30 Kohlenstoffatome aufweisen.

Aus *Crypthecodinium sp.* erhältliche Extrakte sind an sich bekannt. Erfindungsgemäß können sowohl Extrakte von *Crypthecodinium sp.* Wildtyp-Stämmen als auch Extrakte von mutierten oder rekombinierten *Crypthecodinium sp.* Stämmen eingesetzt werden.

Der Begriff "Extrakt aus *Crypthecodinium sp.* " umfasst im vorliegenden Zusammenhang alle Zusaminensetzungen, die durch Extraktion einer Biomasse, vorzugsweise eines Öls, von *Crypthecodinium sp.* mit einem Lösungsmittel, vorzugsweise mit einem organischen und/oder superkritischen Lösungsmittel, insbesondere mit einem organischen Lösungsmittel, gewonnen werden können. Der Einsatz von Lösungsmittelgemischen ist ebenfalls möglich.

Erfindungsgemäß weist der Extrakt eine antioxidative Wirksamkeit auf, die vorzugsweise größer als die der Biomasse ist, aus der der Extrakt gewonnen wird: Es weist daher vorzugsweise einen Peroxidwert auf, der kleiner als der Peroxidwert der ursprünglich eingesetzten, vorzugsweise frisch isolierten, Biomasse ist, aus der der Extrakt gewonnen wird, und vorzugsweise maximal 50,0 %, bevorzugt maximal 25,0 %, zweckmäßigerweise maximal 10,0 %, insbesondere maximal 1,0 % des Peroxidwerts der Biomasse beträgt, aus der der Extrakt gewonnen wird. Der Peroxidwert wird dabei vorzugsweise gemäß AOCS Official Method Cd-3d 63 (American Oil Chemists Society), günstigerweise nach offener Lagerung für 2 Wochen, ermittelt.

Die antioxidative Kapazität des erfindungsgemäßen Extraktes ist vorzugsweise größer 15000 Trolox-Äquivalenten, bevorzugt größer 20000 Trolox-Äquivalenten, zweckmäßigerweise größer 25000 Trolox-Äquivalenten, besonders bevorzugt größer 30000 Trolox-Äquivalenten und insbesondere größer 35000 Trolox-Äquivalenten (µg/ml). Trolox^{®} ist der überlicherweise verwendete Handelsname von 6-Hydroxy-2,5,7,8-tetranethylchroman-2-carbonsäure.

Der Begriff "Biomasse eines Organismus" umfasst erfindungsgemäß sowohl ganze Zellen des Organismus als auch einzelne Zellbestandteile des Organismus.

Der Extrakt aus *Crypthecodinium sp.* wird zweckmäßigerweise erhalten, indem man den Mikroorganismus züchtet, die Biomasse aus der Kultur erntet, aufschließt und den Extrakt isoliert.

Zur Isolierung des Extraktes werden vorzugsweise Extraktionsverfahren mit organischen Lösungsmitteln, insbesondere Hexan, oder mit superkritischen Flüssigkeiten verwendet. Extraktionsverfahren mit organischen Lösungsmitteln werden dabei besonders bevorzugt. Zweckmäßigerweise wird der Extrakt aus der Biomasse durch Perkolation der getrockneten Biomasse mit Hexan extrahiert. Derartige Extraktionen mit organischen Lösungsmitteln sind u. a. in der WO 9737032, in der WO 9743362 und der EP 515460 beschrieben. Eine besonders ausführliche Darstellung findet sich auch im Journal of Dispersion Science and Technology, 10, 561-579, 1989 "Biotechnological Processes for the Production of PUFAs".

Alternativ kann die Extraktion auch ohne Lösungsmittel erfolgen. Ein in diesem Zusammenhang besonders günstiges Verfahren wird in der EP-A-1178118 beschrieben. Bei diesem Verfahren wird ein Lösungsmittel vermieden, indem man eine wässrige Suspension der. Biomasse herstellt und die Ölphase durch Zentrifugation von der wässrigen Phase abtrennt.

Gemäß einer besonders bevorzugten Variante der vorliegenden Erfindung erfolgt die Gewinnung des Extraktes durch reine mechanische Verpressung einer Biomasse aus *Crypthecodinium sp.* und anschließender Extraktion mit mindestens einem organischen oder mindestens einem superkritischen Lösungsmittel, vorzugsweise mit einem organischen Lösungsmittel, insbesondere mit Hexan.

Gemäß einer weiteren besonders bevorzugten Variante der vorliegenden Erfindung erfolgt die Gewinnung des Extraktes destillativ.

Im Rahmen der vorliegenden Erfindung hat es auch sich als ganz besonders vorteilhaft erwiesen, die Biomasse, vorzugsweise mit einem aliphatischen Alkohol mit 1 bis 12 Kohlenstoffatomen, bevorzugt mit 1 bis 6 Kohlenstoffatomen, insbesondere mit 1 bis 4 Kohlenstoffatomen, umzuestern. Dabei hat sich die Verwendung von Methanol und Ethanol, insbesondere von Ethanol ganz besonders bewährt. Die Umesterung erfolgt vorzugsweise unter saurer Katalyse, insbesondere unter Verwendung von Schwefelsäure und/oder Salzsäure. Gemäß einer weiteren, besonders bevorzugten Variante wird die Umesterung enzymatisch erreicht.

Die umgeesterte Biomasse wird anschließend vorzugsweise mit mindestens einem organischen oder superkritischen Lösungsmittel, vorzugsweise mit einem organischen Lösungsmittel, insbesondere mit Hexan, extrahiert. Das Verhältnis des Gesamtvolumens des Lösungsmittels zu dem Volumen der Reaktionsmasse (inklusive dem zugegebenen Wasser) kann auch in einem weiten Bereich variiert werden und ist besonders bevorzugt von 1:3 bis 4:3. Gemäß einer besonders bevorzugten Ausführungsform wird die Mischung mit mehreren Teilen des Lösungsmittels extrahiert, welche am Ende kombiniert werden.

Im Rahmen dieser Ausführungsform wird vorzugsweise ein Hexan-Extrakt einer Biomasse von *Crypthecodinium sp.* als umzuesternde Biomasse eingesetzt, welche dann, wie vorstehend beschrieben, umgeestert wird. Dieser Vorgang dient zur Aufkonzentrierung und Aufteinigung des antioxidativ wirksamen Extraktes. Günstigerweise weist der auf diese Weise aufkonzentrierte und aufgereinigte Extrakt, bezogen auf sein Gesamtgewicht, einen Gehalt an Fettsäuren mit 6 bis 30 Kohlenstoffatomen und an Fettsäureestern, die Fettsäurealkylreste mit 6 bis 30 Kohlenstoffatomen umfassen, von weniger als 20,0 Gew.-%, vorzugsweise von weniger als 10,0 Gew.-%, insbesondere von weniger als 5,0 Gew.-%, auf.

Die Zusammensetzung des Extraktes kann in einem weitem Bereich variieren. Im Rahmen einer ersten, besonders bevorzugten Ausführungsform der vorliegenden Erfindung ist der Extrakt aus *Crypthecodinium sp*. dadurch erhältlich, dass man
i) eine Biomasse von *Crypthecodinium sp*. verseift und
ii) die verseifte Biomasse mit einem Lösungsmittel extrahiert, welches eine Wasserlöslichkeit kleiner 0,1 g Lösungsmittel pro g Wasser bei 25 °C aufweist.

Vorzugsweise wird dabei nach der DGF-Methode F-II 1 (75) vorgegangen.

Das Verseifen der Biomasse kann auf an sich bekannte Weise erfolgen. Besonders bewährt hat sich dabei die Umsetzung der Biomasse mit mindestens einem Alkalimetallhydroxid, vorzugsweise mit NaOH und/oder KOH, insbesondere mit KOH, in alkoholischer Lösung, vorzugsweise in methanolischer und/oder ethanolischer Lösung. Zur Verseifung besonders geeignete Reaktionstemperaturen liegen im Bereich von 25 bis 100 °C.

Die Extraktion der verseiften Produktmischung kann in einem weiten Bereich variieren. Gemäß einer bevorzugten Variante gibt man Wasser zu der Mischung und extrahiert mit einem Lösungsmittel, welches eine Wasserlöslichkeit kleiner 0,1 g Lösungsmittel pro g Wasser bei 25 °C aufweist. Das Verhältnis des Gesamtvolumens des Lösungsmittels zu dem Volumen der Reaktionsmasse (inklusive dem zugegebenen Wasser) kann auch in einem weiten Bereich variiert werden und ist besonders bevorzugt von 1:3 bis 4:3. Gemäß einer besonders bevorzugten Ausführungsform wird die Mischung mit mehreren Teilen des Lösungsmittels extrahiert, welche am Ende kombiniert werden. Erfindungsgemäß besonders geeignete Lösungsmittel schließen die organischen Lösungsmittel Dichlormethan, Diethylether, Methylethylketon, Ethylacetat, Petrolether, Pentan und Hexan sowie die superkritischen Lösungsmittel Propan, Butan und Kohlendixid ein, wobei die organischen Lösungsmittel, vor allem Diethylether und Hexan, insbesondere Diethylether, am meisten bevorzugt werden.

Die Entfernung von verbleibendem Wasser aus der Extraktionslösungsmittelschicht kann beispielsweise durch Waschen der Schicht mit einer Lake (d.h. einer gesättigten Salzlösung), durch Trocknen mit einem Molekularsieb und/oder durch Trocknen mit einem wasserfreien Salz (z.B. Natriumsulfat oder Magnesiumsulfat) erreicht werden.

Nach der Extraktion wird der Extrakt vorzugsweise aufkonzentriert, günstigerweise indem man das Lösungsmittels teilweise oder vollständig verdampft.

Im Rahmen einer weiteren, besonders bevorzugten Ausführungsform der vorliegenden Erfindung ist der Extrakt aus *Crypthecodinium sp*. durch Extraktion einer Biomasse von *Crypthecodinium sp*. mit einem Alkohol mit 1 bis 12, vorzugsweise 1 bis 6, insbesondere 1 bis 4, Kohlenstoffatomen und/oder mit einem Keton mit 3 bis 6, vorzugsweise 3 oder 4, Kohlenstoffatomen erhältlich. Die Extraktion mit einem Alkohol wird dabei gegenüber der Extraktion mit einem Keton bevorzugt. Für die vorliegenden Zwecke ganz besonders geeignete Alkohole sind, jeweils einzeln oder in Mischung, Methanol und Ethanol. Besonders geeignete Ketone umfassen Aceton und/oder Methylethylketon, insbesondere Aceton.

Im Rahmen dieser Ausführungsform wird vorzugsweise ein Hexan-Extrakt einer Biomasse von *Crypthecodinium sp.* als zu extrahierende Biomasse eingesetzt, welche dann mit dem Alkohol und/oder Keton gegenextrahiert wird. Dieser Vorgang dient zur Aufkonzentrierung und Aufreinigung des antioxidativ wirksamen Extraktes. Günstigerweise weist der auf diese Weise aufkonzentrierte und aufgereinigte Extrakt, bezogen auf sein Gesamtgewicht, einen Gehalt an Fettsäuren mit 6 bis 30 Kohlenstoffatomen und Fettsäureestern, die Fettsäurereste mit 6 bis 30 Kohlenstoffatomen umfassen, von weniger als 20,0 Gew.-%, vorzugsweise von weniger als 10,0 Gew.-%, insbesondere von weniger als 5,0 Gew.-%, auf.

Das Verhältnis des Gesamtvolumens des Alkohols oder Ketons zu dem Volumen der Biomasse kann dabei in einem weiten Bereich variiert werden und ist besonders bevorzugt von 3:1 bis 3:4. Gemäß einer besonders bevorzugten Ausführungsform wird die Mischung mit mehreren Teilen des Alkohols oder Ketons extrahiert, welche am Ende kombiniert werden.

Nach der Extraktion wird der Extrakt vorzugsweise aufkonzentriert, günstigerweise indem man das Lösungsmittels teilweise oder vollständig verdampft.

Der auf diese Weise erhältliche Extrakt wird vorzugsweise wiederum mit einem Keton mit 3 bis 6 Kohlenstoffatomen, bevorzugt mit Aceton und/oder Methylethylketon, insbesondere mit Aceton, extrahiert. Das Verhältnis des Gesamtvolumens des Ketons zu dem Volumen des ersten Extraktes kann dabei in einem weiten Bereich variiert werden und ist besonders bevorzugt von 3:1 bis 3:4. Gemäß einer besonders bevorzugten Ausführungsform wird der erste Extrakt mit mehreren Teilen des Ketons extrahiert, welche am Ende kombiniert werden. Nach der Extraktion wird der resultierende zweite Extrakt vorzugsweise aufkonzentriert, günstigerweise indem man das Lösungsmittels teilweise oder vollständig verdampft.

Im Rahmen der vorliegenden Erfindung enthält die Fettsäure-Zusammensetzung weiterhin Bestandteile einer von *Crypthecodinium sp.* verschiedenen Biomasse, vorzugsweise einer Biomasse von *Thraustochytriales,* insbesondere einer Biomasse von *Ulkenia sp..* Von *Crypthecodinium sp.* verschiedene Biomassen sind ebenfalls an sich bekannt. Erfindungsgemäß können sowohl Biomassen von Wildtyp-Stämmen als auch Biomassen von mutierten oder rekombinierten Stämmen eingesetzt werden, die DHA (all-cis 4,7,10,13,16,19 Docosahexaensäure) und/oder DPA (all-cis 4,7,10,13,16 Docosapentaensäure) effizient herstellen. Derartige mutierte oder rekombinierte Stämme schließen Mikroorganismen ein, die, verglichen mit dem Prozentsatz des original Wildtyp-Stamms, unter Verwendung desselben Substrates, einen höheren Prozentsatz DHA und/oder DPA in Fetten, und/oder verglichen mit der durch den original Wildtyp-Stamm hergestellten Menge, unter Verwendung desselben Substrates, eine höhere Gesamtmenge der Lipide enthalten.

Gemäß einer besonders bevorzugten Ausführungsform der vorliegenden Erfindung enthält die erfindungsgemäße Fettsäure-Zusammensetzung einen Extrakt der von *Crypthecodinium sp.* verschiedenen Biomasse. Der Extrakt wird dabei zweckmäßigerweise erhalten, indem man den betreffenden Mikroorganismus züchtet, die Biomasse aus der Kultur erntet, aufschließt und den Extrakt isoliert. Ein in diesem Zusammenhang ganz besonders günstiges Verfahren wird in der WO 03/033631 A1 beschrieben, auf deren Offenbarung hiermit explizit bezug genommen wird.

Zur Isolierung des Extraktes werden vorzugsweise Extraktionsverfahren mit organischen Lösungsmitteln, insbesondere Hexan, oder mit superkritischen Flüssigkeiten verwendet. Zweckmäßigerweise wird der Extrakt aus der Biomasse durch Perkolation der getrockneten Biomasse mit Hexan extrahiert. Derartige Extraktionen mit organischen Lösungsmitteln sind u. a. in der WO 9737032, in der WO 9743362 und der EP 515460 beschrieben. Eine besonders ausführliche Darstellung findet sich auch im Journal of Dispersion Science and Technology, 10, 561-579, 1989 "Biotechnological Processes for the Production of PUFAs".

Alternativ kann die Extraktion auch ohne Lösungsmittel erfolgen. Ein in diesem Zusammenhang besonders günstiges Verfahren wird in der EP-A-1178118 beschrieben. Bei diesem Verfahren wird ein Lösungsmittel vermieden, indem man eine wässrige Suspension der Biomasse herstellt und die Ölphase durch Zentrifugation von der wässrigen Phase abtrennt.

Gemäß einer besonders bevorzugten Variante der vorliegenden Erfindung erfolgt die Gewinnung des Extraktes durch reine mechanische Verpressung einer von *Crypthecodinium sp.* verschiedenen Biomasse und anschließender Extraktion mit mindestens einem organischen oder superkritischen Lösungsmittel, vorzugsweise mit mindestens einem organischen Lösungsmittel, insbesondere mit Hexan.

Im Rahmen der vorliegenden Erfindung hat es sich auch als ganz besonders vorteilhaft erwiesen, die Biomasse, vorzugsweise mit einem aliphatischen Alkohol mit 1 bis 12 Kohlenstoffatomen, bevorzugt mit 1 bis 6 Kohlenstoffatomen, insbesondere mit 1 bis 4 Kohlenstoffatomen, umzuestern. Dabei hat sich die Verwendung von Methanol und Ethanol, insbesondere von Ethanol ganz besonders bewährt. Die Umesterung erfolgt vorzugsweise unter saurer Katalyse, insbesondere unter Verwendung von Schwefelsäure und/oder Salzsäure. Die umgeesterte Biomasse wird anschließend vorzugsweise mit einem organischen Lösungsmittel, insbesondere mit Hexan, extrahiert. Das Verhältnis des Gesamtvolumens des Lösungsmittels zu dem Volumen der Reaktionsmasse (inklusive dem zugegebenen Wasser) kann auch in einem weiten Bereich variiert werden und ist besonders bevorzugt von 1:3 bis 4:3. Gemäß einer besonders bevorzugten Ausführungsform wird die Mischung mit mehreren Teilen des Lösungsmittels extrahiert, welche am Ende kombiniert werden.

Die Zusammensetzung der Biomasse kann in einem weitem Bereich variieren. Vorzugsweise enthält die von *Crypthecodinium sp.* verschiedene Biomasse mindestens eine mehrfach ungesättigte Fettsäure und/oder mindestens einen Fettsäureester zweckmäßigerweise einen Fettsäurealkylester, bevorzugt ein Glycerid, insbesondere ein Triglycerid, welcher mindestens einen mehrfach ungesättigten Fettsäure-Rest umfasst, der vorzugsweise 6 bis 30 Kohlenstoffatome aufweist. Gemäß einer besonders bevorzugten Ausführungsform sind mindestens 10 %, besonders bevorzugt mindestens 25 % und insbesondere mindestens 30 % der Fettsäuren und/oder der Fettsäure-Reste in der Biomasse DHA bzw. DHA-Reste.

Ein "Glycerid" ist, so wie der Ausdruck hierin verwendet wird, ein Ester von Glycerin und mindestens einer Fettsäure, wobei eine bis drei Hydroxylgruppen des Glycerins mit einem oder mehreren Fettsäure-Resten verestert wurden. Wenn mehrere Fettsäure-Reste vorliegen, können die Fettsäure-Reste gleich oder verschieden sein.

Bei vielen geeigneten Ausgangsmaterialien sind der Hauptanteil der Glyceride Triglyceride, d. h. Ester von drei Fettsäure-Resten und Glycerin. Dabei kann jeder Fettsäure-Rest entweder gesättigt (d.h. alle Bindungen zwischen den KohlenstoffAtomen sind Einfachbindungen) oder ungesättigt (d.h. es gibt mindestens eine Kohlenstoff Kohlenstoff Doppel- oder -Dreifachbindung) sein. Die Art der ungesättigten Fettsäure-Reste werden hierin gelegentlich mit einem ω gekennzeichnet. Diese Nummer gibt die Position der ersten Doppelbindung an, wenn man ausgehend von der terminalen Methylgruppe der Fettsäure oder des Fettsäure-Restes zählt.

Die relativen Anteile der einzelnen Komponenten der erfindungsgemäßen Fettsäure-Zusammensetzung können grundsätzlich frei gewählt und auf die jeweilige Anwendung abgestimmt werden. Im Rahmen der vorliegenden Erfindung hat es sich jedoch als ganz besonders günstig erwiesen, dass die Fettsäure-Zusammensetzung, jeweils bezogen auf ihr Gesamtgewicht, 0,1 bis 50,0 Gew.-%, vorzugsweise 0,1 bis 25,0 Gew.-%, günstigerweise 0,2 bis 10,0 Gew.-%, insbesondere 0,5 bis 5,0 Gew.-%, des antioxidativ wirksamen Extraktes aus *Crypthecodinium sp.* und 50,0 bis 99,9 Gew.-% , vorzugsweise 75,0 bis 99,9 Gew.-%, günstigerweise 90,0, bis 99,8 Gew.-%, insbesondere 95,0 bis 99,5 Gew.- %, Bestandteile einer von *Crypthecodinium sp.* verschiedenen Biomasse enthält, wobei die zuvor genannten relativen Anteile zusammengenommen vorzugsweise 100,0 Gew.-% ergeben.

Die erfindungsgemäße Fettsäure-Zusammensetzung weist einen vergleichsweise hohen Anteil an mehrfach ungesättigten Fettsäuren auf und enthält, jeweils bezogen auf ihr Gesamtgewicht, vorzugsweise mindestens 10,0 Gew.-%, günstigerweise mindestens 25,0 Gew.-%, bevorzugt mindestens 50,0 Gew.-%, insbesondere mindestens 70,0 Gew.-%, Docosahexaensäure (all-cis 4,7,10,13,16,19 Docosahexaensäure) und/oder Docosahexaensäurealkylester (all-cis 4,7,10,13,16,19 Docosahexaensäurealkylester), bevorzugt Docosahexaensäure, Docosahexaensäuremethylester und/oder Docosahexaensäureethylester.

Die erfindungsgemäße Fettsäure-Zusammensetzung zeichnet sich gegenüber herkömmlich stabilisierten Fettsäure-Zusammensetzungen durch eine höhere oxidative Stabilität aus. Die Zugabe von an sich bekannten Antioxidantien, wie beispielsweise α-, β-, γ- und/oder δ-Tocopherol, ist daher nicht unbedingt erforderlich. Dementsprechend enthält die erfindungsgemäße Fettsäure-Zusammensetzung gemäß einer ersten bevorzugten Ausführungsform keine weiteren Antioxidantien.

Da jedoch die antioxidative Stabilität der erfindungsgemäßen Fettsäure-Zusammensetzung oftmals durch die zusätzliche Zugabe von Antioxidantien noch weiter gesteigert werden kann, enthält die Fettsäure-Zusammensetzung gemäß einer ganz besonders bevorzugten Ausführungsform der Erfindung mindestens ein, vorzugsweise synergistisch wirkendes, Antioxidans, vorzugsweise mindestens ein Tocotrienol, α-, β-, γ- und/oder δ-Tocopherol, günstigerweise α-, β-, γ- und/oder δ-Tocopherol, insbesondere α-, β-, γ- und/oder δ-Tocopherol und Ascorbylpalmitat, wobei der relative Anteil dieser Komponente vorzugsweise 0,01 bis 5,0 Gew.-%, insbesondere 0,05 bis 0,5 Gew.-%, jeweils bezogen auf das Gesamtgewicht der Fettsäure-Zusammensetzung, beträgt.

Die Herstellung der erfindungsgemäßen Fettsäure-Zusammensetzung erfolgt auf an sich bekannte Weise, vorzugsweise durch Mischen der entsprechenden Komponenten. Dabei hat es sich als ganz besonders vorteilhaft erwiesen, der antioxidativ wirksame Extrakt aus *Crypthecodinium sp.* und die Bestandteile der von *Crypthecodinium sp.* verschiedenen Biomasse getrennt voneinander in einem Lösungsmittel, vorzugsweise Petrolether, Hexan, Pentan, Ethanol, Methanol, Acetonitril, Dichlormethan, Methylethylketon, Diethylether und/oder Ethylacetat, günstigerweise Hexan und/oder Diethylether, insbesondere Diethylether, zu lösen, die Lösungen dann miteinander zu mischen und das Lösungsmittel anschließend, vorzugsweise durch verdampfen, zu entfernen.

Gemäß einer weiteren bevorzugten Ausführungsform der Erfindung werden die Komponenten ohne Zusatz von Lösungsmittel gemischt, wobei ggfs. höhere Temperaturen, vorzugsweise im Bereich von 25 °C bis 80 °C, insbesondere im Bereich von 25 °C bis 60 °C, verwendet werden.

Mögliche Anwendungsgebiete der erfindungsgemäßen Fettsäure-Zusammensetzung sind dem Fachmann unmittelbar offensichtlich. Sie eignen sich insbesondere für alle Anwendungen, die für PUFAs und PUFA-Ester vorgezeichnet sind. Dabei kann die erfindungsgemäße Fettsäuren-Zusammensetzung meist direkt eingesetzt werden. Für manche Anwendungen ist es jedoch nötig, den oder die Fettsäure-Ester in der flüssigen Phase vorher zu verseifen. Dies kann beispielsweise durch Umsetzung mit KOH in Ethanol und anschliessendem Ansäuern mit einer anorganischen oder organischen Säure erreicht werden.

Die erfindungsgemäße Fettsäure-Zusammensetzung wird insbesondere als Wirkstoff oder Bestandteil in pharmazeutischen Zusammensetzungen, als Bestandteil in kosmetischen Zubereitungen, als Lebensmittelzusatzstoff, als Lebensmittelzutat, als Bestandteil funktioneller Nahrungsmittel und zur Herstellung höher konzentrierter PUFA-Folgeprodukte, wie Ester und Säuren, eingesetzt.

Nachfolgend wird die Erfindung durch Beispiele eingehender erläutert, ohne dass hierdurch eine Beschränkung des Erfindungsgedankens erfolgen soll.

Es wurden die Induktionszeit, die Peroxidwerte und/oder die antioxidative Kapazität von folgenden Fettsäure-Zusammensetzungen bestimmt:

### Kontrolle 1

Ein wie in Yokochi et al., Appl. Microb. Biotechnol., (1998), 49, S. 72-76 beschrieben, hergestelltes "DHA-haltiges Öl" wurde verwendet. Dieses wurden nach allgemein bekannten Verfahrensschritten einer vollständigen Raffination unterzogen. Im folgenden wird dieses Öl kurz als "DHA-haltiges Öl" bezeichnet.

### Kontrolle 2-17

"DHA-haltiges Öl" + die Tabelle 1 angegebenen Mengen Ascorbylpalmitat und/oder Tocopherolgemisch (zugegeben 0,14%. ^{®}Coviox T70; natürliches Tocopherolgemisch).

### Beispiel 1

Die Extraktgewinnung wurde nach DGF-Methode F-II 1 (75) durchgeführt.

5,02 g *Crypthecodinium cohnii*-Rohöl (Hexan-Extrakt) wurden in einen 250 ml Rundkolben eingewogen und mit 20 mg Pyrogallol, 40 ml Methanol, 10 ml 60%iger Kalilauge (g/v) und 3 Siedesteinen versetzt. Im 80 °C heißen Wasserbad wurde die Probe 20 Minuten lang unter Rückfluß und leichtem Stickstoffstrom verseift. Nach dem Abkühlen wurde die Seifenlösung 3-mal mit 40 ml bidestilliertem Wasser und 2-mal mit 50 ml Diethylether in einen 500 ml Scheidetrichter überspült.

Mit dem Diethylether erfolgte unter vorsichtigem Schwenken eine erste Extraktion. Die wässrige Phase wurde in ein 600 ml Becherglas abgelassen. Die Diethyletherphase wurde mit 40 ml bidestilliertem Wasser nachgewaschen, das Wasser zur wässrigen Phase abgelassen. Die Diethyletherphase wurde in einen 1000 ml Rundkolben abgelassen. Die wässrige Phase wurde noch viermal, wie beschrieben, behandelt (Diethylether zugegeben, Extraktion usw.), bis sie farblos war. Die vereinigten Diethylphasen wurden am Rotationsverdampfer eingeengt, mittels Ölpumpe getrocknet und ausgewogen. Es wurden 921 mg Extrakt gewonnen.

Dieser wurde mit 4 g "DHA-haltigem Öl" (Kontrolle 5; enthält 0,1 % Tocopherol) versetzt und unter Zuhilfenahme von 10 ml Diethylether gut gemischt. Nach Entfernung des Diethylethers wurde ein oranges Öl erhalten.

### Beispiel 2

Es wurden 41,9 g *Crypthecodinium* cohnii-Rohöl in einen 500 ml Rundkolben eingewogen, mit 120 ml Methanol und einem Magnetrührstab versetzt. Der Ansatz wurde 3 Stunden auf dem Magnetrührer kräftig gerührt. Die obere Methanolphase wurde in einen 250 ml Rundkolben abdekantiert. Der Ölansatz wurde nochmals mit 100 ml Methanol versetzt und eine Stunde lang nachgewaschen. Das Öl-Methanol-Gemisch wurde in einen 100 ml Scheidtrichter gegeben und die Methanolphase zu der bereits Vorhandenen überführt. Diese wurde am Rotationsverdamfer eingeengt und mittels Ölpumpe getrocknet. Es wurden 760 mg Extrakt gewonnen. Ein "DHA-haltiges Öl" (Kontrolle 5; enthält 0,1% Tocopherol) wurde mit 2 Gew.-% des Extraktes versetzt und gut vermischt.

### Beispiel 3

Erhalten wie die Fettsäure-Zusammensetzung aus Beispiel 2, außer dass das eingesetzte "DHA-haltige Öl" (Kontrolle 1) mit 4 Gew.-% des Extraktes versetzt und gut vermischt wurde.

### Beispiel 4

Eine *Crypthecodinium cohii*-Biotrockenmasse wurde direkt mit Methanol extrahiert, wobei auch ein großer Anteil Phospholipide mit extrahiert wurde, was zu einem sehr zähen Produkt führte.

Ein "DHA-haltiges Öl" (Kontrolle 1) wurde mit 4 Gew.-% des Extraktes versetzt und gut vermischt.

### Beispiel 5

Eine *Crypthecodinium cohnii*-Biotrockemnasse wurde direkt mit Methanol extrahiert, wobei auch ein großer Anteil Phospholipide mit extrahiert wurde, was zu einem sehr zähen Produkt führte. Zur Entfernung dieser Verbindungen wurde der Extrakt nochmals mit Aceton gewaschen und die im Aceton löslichen Bestandteile bildeten den *Crypthecodinium cohnii*-Acetonextrakt.

Ein "DHA-haltiges Öl" (Kontrolle 1) wurde mit 4 Gew.-% des Acetonextraktes versetzt und gut vermischt.

### Beispiel 6

Erhalten wie die Fettsäure-Zusammensetzung aus Beispiel 2, außer dass das eingesetzte "DHA-haltige Öl" (Kontrolle 5) mit 4 Gew.-% des Extraktes versetzt und gut vermischt wurde.

### Rancimatbestimmung

| | | |
|---|---|---|
| Gerät: | | 743 Rancimat |
| Hersteller: | | Metrohm |
| Geräteeinstellungen: | | |
| | Methode: | (analog zu AOCS Methode Cd12b-92) |
| | Temperatur: | 80°C |
| | Gasfluss: | 20L/h |
| | Stoppkriterium: | Endpunkt |

### Durchführung und Messprinzip:

Das zu vermessende Öl (3 g) wird in ein Reaktionsgefäß eingewogen, in den Heizblock gestellt und einer definierten Temperatur und einem Luftstrom ausgesetzt. Dabei entstehen flüchtige Oxidationsprodukte, wie Ameisensäure, die über ein Luftrohr in das Messgefäß transferiert werden, in dem mit der Leitfähigkeitselektrode in destilliertem Wasser die Leitfähigkeit gemessen wird. Diese wird über die Zeit bis zum Endpunkt aufgezeichnet. Von dieser Kurve wird automatisch die zweite Ableitung gebildet, die beim Sattelpunkt ihr Maximum hat. Die Zeit bis zum Sattelpunkt wird als Induktionszeit bezeichnet.

Je höher die Stabilität der jeweiligen Probe ist, desto höher ist auch die Induktionszeit. Demzufolge können durch den Vergleich der gemessenen Induktionszeiten Rückschlüsse über den anti-/oxidativen Status einer Probe gezogen werden sowie die Wirksamkeit von Antioxidantien effektiv miteinander verglichen werden.

Für die vorstehend aufgeführten Materialien wurden die in Tabelle 1 zusammengefaßten Induktionszeiten gemessen.

**Tabelle 1: Induktionszeiten nach dem Rancimattest**

| **Probe** | **Zusatz** | **Induktionszeit (h)** |
|---|---|---|
| Kontrolle 1 | --- | 1,1 |
| Kontrolle 2 | 0,01 Gew.-%Toc | 1,9 |
| Kontrolle 3 | 0,025 Gew.-% Toc | 3,7 |
| Kontrolle 4 | 0,05 Gew.-%Toc | 5,5 |
| Kontrolle 5 | 0,1 Gew.-%Toc | 5,7 |
| Kontrolle 6 | 0,15 Gew.-% Toc | 6,8 |
| Kontrolle 7 | 0,2 Gew.-%Toc | 7,7 |
| Kontrolle 8 | 0,5 Gew.-%Toc | 7,0 |
| Kontrolle 9 | 1,0 Gew.-%Toc | 7,5 |
| Kontrolle 10 | 2,0 Gew.-% Toc | 6,6 |
| Kontrolle 11 | 0,025 Gew.-%Toc + 0,025 Gew.-%AP | 4,3 |
| Kontrolle 12 | 0,1 Gew.-%Toc + 0,025 Gew.-%AP | 9,0 |
| Kontrolle 13 | 0,1 Gew.-%Toc + 0,5 Gew.-%AP | 8,5 |
| Kontrolle 14 | 0,1 Gew.-%Toc + 0,1 Gew.-%AP | 7,4 |
| Kontrolle 15 | 0,2 Gew.-%Toc + 0,05 Gew.-%AP | 11,6 |
| Kontrolle 16 | 0,2 Gew.-%Toc + 0,1 Gew.-%AP | 10,6 |
| Kontrolle 17 | 0,2 Gew.-%Toc + 0,2 Gew.-%AP | 7,0 |
| Beispiel 1 | 18,7 Gew.-% UVA + 0,1 Gew.-% Toc | 17,9 |
| Beispiel 2 | 2,0 Gew.-% MeOH-Extr. + 0,1 Gew.-% Toc | 14,3 |
| Beispiel 6 | 4,0 Gew.-% MeOH-Extr. + 0,1 Gew.-% Toc | 17,6 |
| Beispiel 5 | 4 Gew.-% Ace-Extr. | 41,5 |

| | | |
|---|---|---|
| AP: Ascorbylpalmitat Toc: Tocopherolgemisch uvA: unverseifbare Anteile (s.o.) MeOH-Extr.: MeOH-Extrakt (s.o.) Ace-Extr.-Aceton-Extrakt (s.o.) | | |

### Bestimmung der Peroxidwerte

Die vorstehenden Materialien wurden für vorbestimmte Zeiten in offenen 100 ml Erlenmeyerkolben im Dunkeln bei Raumtemperatur gelagert und anschließend hinsichtlich ihrer Peroxidwerte untersucht. Die Bestimmung der Peroxidwerte erfolgte gemäß AOCS Official Method Cd-3d 63 (American Oil Chemists Society). Die erhaltenen Ergebnisse werden in Tabelle 2 zusammengefaßt. Sie zeigen, dass sich durch Verwendung des Methanol-Extraktes (Beispiel 3) die antioxidative Stabilität gegenüber dem "DHA-haltigem Öl" ohne zusätzlichen Stabilisator (Kontrolle 1) oder dem konventionell stabilisierten "DHA-haltigem Öl" (Kontrolle 2) deutlich steigern lässt. Dabei lässt sich die antioxidative Stabilität durch die zusätzliche Zugabe von Tocopherol noch weiter erhöhen.

**Tabelle 2: Peroxidwerte nach offener Lagerung**

| **Lagerzeit** | **Kontrolle 1** | **Kontrolle 2** | **Beispiel 3** | **Beispiel 6** |
|---|---|---|---|---|
| 0 Tage | 0,5 | 0,7 | 0,6 | 0,6 |
| 2 Tage | 3,0 | 1,2 | 1,4 | 1,3 |
| 7 Tage | 9,5 | 3,0 | 1,9 | 2,7 |
| 14 Tage | 17,8 | 4,2 | 2,8 . | 2,1 |
| 21 Tage | 74,5 | 24,9 | 3,1 | 4,2 |

### Bestimmung, der antioxidativen Kapazität

Von den Kontrollen 1 und 5 sowie vom Beispiel 5 wurde die antioxidative Kapazität wie folgt bestimmt.

### Methode:

Die Proben wurden nach der Photochem-Methode vermessen. Das Photochem^{®} arbeitet nach der Photochemolumineszenz (PCL)-Methode. Mit Hilfe eines Photosensitizers werden Superoxidanionen-Radikale erzeugt, die über ihre Reaktion mit einer chemoluminogenen Substanz (z.B. Luminol) und der Messung des dadurch entstehenden Lichtes nachgewiesen werden. Je mehr Radikalfänger (Antioxidantien) in der Probe enthalten sind, desto stärker wird die Intensität der Photochemolumineszenz konzentrationsabhängig abgeschwächt. Die Resultate werden in äquivalenten Konzentrationseinheiten von Trolox angegeben. Das Gerät arbeitet mit standardisierten Kits für die Messung der integralen antioxidativen Kapazität einzelner Antioxidantien und der Superoxiddismutase.

Für die Bestimmung der Trolox-Äquivalente wurden die Proben mit n-Hexan verdünnt und direkt für die Messung eingesetzt.

Die erhaltenen Ergebnisse werden in Tabelle 3 zusammengefasst.

**Tabelle 3: Antioxidative Kapazität einiger Proben**

| **Probe** | **Antioxidative Kapazität Trolox-Äquivalente (µg/ml)** |
|---|---|
| Kontrolle 1 | 20,4 |
| Kontrolle 5 | 328 |
| Beispiel 5 | 1143 |

Es ist zu erkennen, dass die erfindungsgemäßen Beispiele vergleichsweise hohe antioxidative Kapazitäten aufweisen. Dabei ist zu beachten, dass die Menge des zugesetzten Extraktes nicht gleichzusetzen ist mit der Menge der antioxidativ wirksamen Menge im Gemisch. So sind weitere Aufreinigungen möglich und führen zu antioxidativ noch wirksameren Extrakten. Selbstverständlich beinhaltet der Schutzumfang noch stärker aufgereinigte Konzentrate bis hin zu den antioxidativ wirksamen Verbindungen.

## Patentansprüche

1. Verwendung eines Extraktes aus *Crypthecodinium sp*. mit antioxidativer Wirkung für die antioxidative Stabilisierung von Fettsäure-Zusammensetzungen aus einer von *Crypthecodinium sp*. verschiedenen Biomasse.

2. Verwendung nach Anspruch 1, wobei der Extrakt eine antioxidative Kapazität von größer 25000 Trolox-Äquivalenten aufweist.

3. Verwendung nach Anspruch 2, **dadurch gekennzeichnet, dass** der Extrakt dadurch erhältlich ist, dass man
i) eine Biomasse von *Crypthecodinium sp*. verseift und
ii) die verseifte Biomasse mit einem Lösungsmittel extrahiert, welches eine Wasserlöslichkeit kleiner 0,1 g Lösungsmittel pro g Wasser bei 25 °C aufweist.

4. Verwendung nach Anspruch 3, **dadurch gekennzeichnet, dass** der Extrakt durch Extraktion einer verseiften Biomasse aus *Crypthecodinium sp*. mit Hexan, Pentan, Ethylacetat, Diethylether, Dichlormethan, Dimethylethylketon, und/oder überkritischem Kohlendioxid erhältlich ist.

5. Verwendung nach Anspruch 2, **dadurch gekennzeichnet, dass** der Extrakt durch Extraktion einer Biomasse von *Crypthecodinium sp*. mit einem Alkohol mit 1 bis 12 Kohlenstoffatomen und/oder mit einem Keton mit 3 bis 6 Kohlenstoffatomen erhältlich ist.

6. Verwendung nach Anspruch 5, **dadurch gekennzeichnet, dass** der Extrakt durch Extraktion einer Biomasse von *Crypthecodinium sp*. mit Methanol, Isopropanol, Aceton und/oder Ethanol erhältlich ist.

7. Verwendung nach Anspruch 5 oder 6, **dadurch gekennzeichnet, dass** der Extrakt durch Extraktion einer Biomasse von *Crypthecodinium sp*. mit einem Alkohol mit 1 bis 12 Kohlenstoffatomen und anschließender Extraktion mit einem Keton erhältlich ist.

8. Verwendung nach mindestens einem der vorangehenden Ansprüche, **dadurch gekennzeichnet, dass** der Extrakt ein Extrakt aus *Crypthecodinium cohnii* ist.

9. Verwendung nach mindestens einem der vorangehenden Ansprüche, **dadurch gekennzeichnet, dass** der Extrakt durch ein Verfahren erhältlich ist, bei welchem man eine Biomasse von *Crypthecodinium sp*. umestert.

10. Verwendung nach mindestens einem der vorangehenden Ansprüche, **dadurch gekennzeichnet, dass** der Extrakt durch ein Verfahren erhältlich ist, bei welchem man eine Biomasse von *Crypthecodinium sp*. mechanisch extrahiert.

11. Antioxidativ stabilisierte Fettsäure-Zusammensetzung, welche mindestens eine ungesättigte Fettsäure enthält, **dadurch gekennzeichnet, dass** die Fettsäure-Zusammensetzung mindestens einen Extrakt aus *Crypthecodinium sp*. mit antioxidativer Wirkung und Bestandteile einer von *Crypthecodinium sp*. verschiedenen Biomasse enthält, wobei die Fettsäure-Zusammensetzung keine weiteren Antioxidantien enthält.

12. Antioxidativ stabilisierte Fettsäure-Zusammensetzung nach Anspruch 11, **dadurch gekennzeichnet, dass** sie Bestandteile einer Biomasse von *Thraustochytriales* enthält.

13. Antioxidativ stabilisierte Fettsäure-Zusammensetzung nach Anspruch 12, **dadurch gekennzeichnet, dass** sie Bestandteile einer Biomasse von *Ulkenia sp*. enthält.

14. Antioxidativ stabilisierte Fettsäure-Zusammensetzung nach mindestens einem der Ansprüche 11 bis 13, **dadurch gekennzeichnet, dass** die Bestandteile der von *Crypthecodinium sp*. verschiedenen Biomasse durch ein Verfahren erhältlich sind, bei welchem man eine von *Crypthecodinium sp*. verschiedene Biomasse umestert.

15. Antioxidativ stabilisierte Fettsäure-Zusammensetzung nach mindestens einem der Ansprüche 11 bis 14, **dadurch gekennzeichnet, dass** sie, jeweils bezogen auf das Gesamtgewicht der Fettsäure-Zusammensetzung, 0,1 bis 50,0 Gew.-% mindestens eines Extraktes nach mindestens einem der Ansprüche 1 bis 10 und 50,0 bis 99,9 Gew.-% Bestandteile einer von *Crypthecodinium sp*. verschiedenen Biomasse enthält.

16. Antioxidativ stabilisierte Fettsäure-Zusammensetzung nach mindestens einem der vorangehenden Ansprüche 11 bis 15, **dadurch gekennzeichnet, dass** sie, bezogen auf ihr Gesamtgewicht, mindestens 25,0 Gew.-% Docosahexaensäure und/oder Docosahexaensäurealkylester enthält.

17. Verfahren zur Herstellung einer antioxidativ stabilisierten Fettsäure-Zusammensetzung, **dadurch gekennzeichnet, dass** man mindestens einen Extrakt aus *Crypthecodinium sp*. mit antioxidativer Wirkung und Bestandteile einer von *Crypthecodinium sp*. verschiedenen Biomasse miteinander mischt, wobei die Fettsäure-Zusammensetzung keine weiteren Antioxidantien enthält.

18. Verwendung einer Fettsäure-Zusammensetzung gemäß mindestens einem der Ansprüche 11 bis 16 als Wirkstoff oder Bestandteil in pharmazeutischen Zusammensetzungen.

19. Verwendung einer Fettsäure-Zusammensetzung gemäß mindestens einem der Ansprüche 11 bis 16 als Bestandteil in kosmetischen Zubereitungen.

20. Verwendung einer Fettsäure-Zusammensetzung gemäß mindestens einem der Ansprüche 11 bis 16 als Lebensmittelzusatzstoff und/oder als Lebensmittelzutat.

21. Verwendung einer Fettsäure-Zusammensetzung gemäß mindestens einem der Ansprüche 11 bis 16 als Bestandteil von Tierfutter.

## Claims

1. The use of an extract from *Crypthecodinium sp*. having antioxidative activity for the antioxidative stabilization of fatty acid compositions of a biomass different from *Crypthecodinium sp.*

2. The use as claimed in claim 1, wherein the extract has an antioxidative capacity of greater than 25 000 Trolox equivalents.

3. The use as claimed in claim 2, **characterized in that** the extract is obtainable by
i) saponifying a biomass of *Crypthecodinium sp*. and
ii) extracting the saponified biomass with a solvent which has a water solubility less than 0.1 g of solvent per g of water at 25°C.

4. The use as claimed in claim 3, **characterized in that** the extract is obtainable by extracting a saponified biomass from *Crypthecodinium sp*. with hexane, pentane, ethyl acetate, diethyl ether, dichloromethane, dimethyl ethyl ketone, and/or supercritical carbon dioxide.

5. The use as claimed in claim 2, **characterized in that** the extract is obtainable by extracting a biomass of *Crypthecodinium sp*. with an alcohol having 1 to 12 carbon atoms and/or with a ketone having 3 to 6 carbon atoms.

6. The use as claimed in claim 5, **characterized in that** the extract is obtainable by extracting a biomass of *Crypthecodinium sp*. with methanol, isopropanol, acetone and/or ethanol.

7. The use as claimed in claim 5 or 6, **characterized in that** the extract is obtainable by extracting a biomass of *Crypthecodinium sp*. with an alcohol having 1 to 12 carbon atoms and subsequent extraction with a ketone.

8. The use as claimed in at least one of the preceding claims, **characterized in that** the extract is an extract from *Crypthecodinium cohnii.*

9. The use as claimed in at least one of the preceding claims, **characterized in that** the extract is obtainable by a method in which a biomass of *Crypthecodinium sp*. is transesterified.

10. The use as claimed in at least one of the preceding claims, **characterized in that** the extract is obtainable by a method in which a biomass of *Crypthecodinium* sp. is mechanically extracted.

11. An antioxidatively stabilized fatty acid composition which contains at least one unsaturated fatty acid, **characterized in that** the fatty acid composition contains at least one extract from *Crypthecodinium sp*. having antioxidative activity and components of a biomass different from *Crypthecodinium sp*., wherein the fatty acid composition does not contain further antioxidants

12. The antioxidatively stabilized fatty acid composition as claimed in claim 11, **characterized in that** it contains components of a biomass of *Thraustochytriales*.

13. The antioxidatively stabilized fatty acid composition as claimed in claim 12, **characterized in that** it contains components of a biomass of *Ulkenia sp*.

14. The antioxidatively stabilized fatty acid composition as claimed in at least one of claims 11 to 13, **characterized in that** the components of the biomass different from *Crypthecodinium sp*. are obtainable by a method in which a biomass different from *Crypthecodinium sp*. is transesterified.

15. The antioxidatively stabilized fatty acid composition as claimed in at least one of claims 11 to 14, **characterized in that** it, in each case based on the total weight of the fatty acid composition, contains 0.1 to 50.0% by weight of at least one extract as claimed in at least one of claims 1 to 10 and 50.0 to 99.9% by weight of components of a biomass different from *Crypthecodinium sp.*

16. The antioxidatively stabilized fatty acid composition as claimed in at least one of the preceding claims 11 to 15, **characterized in that** it, based on its total weight, contains at least 25.0% by weight docosahexaenoic acid and/or docosahexaenoic acid alkyl ester.

17. A process for producing an antioxidatively stabilized fatty acid composition, **characterized in that** at least one extract from *Crypthecodinium sp.* having antioxidative activity and components of a biomass different from *Crypthecodinium sp.* are mixed with one another, wherein the fatty acid composition does not contain further antioxidants.

18. The use of a fatty acid composition as claimed in at least one of claims 11 to 16 as active ingredient or component in pharmaceutical compositions.

19. The use of a fatty acid composition as claimed in at least one of claims 11 to 16 as component in cosmetics preparations.

20. The use of a fatty acid composition as claimed in at least one of claims 11 to 16 as food additive and/or as food ingredient.

21. The use of a fatty acid composition as claimed in at least one of claims 11 to 16 as component of animal feed.

## Revendications

1. Utilisation d'un extrait de crypthecodinium sp. avec un effet antioxydant pour la stabilisation anti-oxydative des composé d'acide gras d'une biomasse différente de celle du crypthecodinium sp.

2. Utilisation selon la revendications 1, au moment de quoi l'extrait démontre une capacité anti-oxydante majeure de 25.000 équivalents trolox.

3. Utilisation selon la revendication 2, **caractérisée en ce que** l'extrait est obtenu en :
i) saponifiant une biomasse de crypthecodinium sp. et
ii) en extrayant la biomasse saponifiée avec un solvant ayant une solubilité dans l'eau aussi basse que au 0,1 g de solvant par g d'eau à 25 °C.

4. Utilisation selon la revendication 3, **caractérisée en ce que** l'extrait peut être obtenu par l'extraction d'une biomasse saponifiée de crypthecodinium sp. avec hexane, pentane, acétate éthylique, diéthyléther, dichlorométhane, diméthyle éthyle cétone, et/ou dioxyde de carbone hypercritique.

5. Utilisation selon la revendication 2, **caractérisée en ce que** l'extrait peut être obtenu par extraction d'une biomasse de crypthecodinium sp. avec un alcool contenant de 1 à 12 atomes de carbone et/ou avec un cétone contenant de 3 à 6 atomes de carbone.

6. Utilisation selon la revendication 5, **caractérisée en ce que** l'extrait peut être obtenu par extraction d'une biomasse de crypthecodinium sp. avec méthanol, isopropanol, acétone et/ou éthanol.

7. Utilisation selon les revendications 5 ou 6, **caractérisée en ce que** l'extrait peut être obtenu par extraction d'une biomasse de crypthecodinium sp. avec un alcool contenant de 1 à 12 atomes de carbone et ensuite par extraction avec un cétone.

8. Utilisation selon au moins une des revendications précédentes, **caractérisée en ce que** l'extrait est un extrait de crypthecodinium cohnii.

9. Utilisation selon une des revendications précédentes, **caractérisée en ce que** l'extrait peut être obtenu par un procédé de transestérification d'une biomasse de crypthecodinium sp.

10. Utilisation selon au moins une des revendications précédentes, **caractérisée en ce que** l'extrait peut être obtenu par un procédée mécanique d'extraction d'une biomasse d'un crypthecodinium sp.

11. Composé anti-oxydatif et stabilisant d'acide gras contenant au moins un acide gras insaturé, **caractérisé en ce que** la composition d'acide gras contient au moins un extrait de crypthecodinium sp. avec effet antioxydant et des composants d'une biomasse différente du crypthecodinium sp. sans que la composition d'acide gras ne contienne d'autres antioxydants.

12. Composé anti-oxydatif et stabilisant d'acide gras selon la revendication 11, **caractérisé en ce qu'**elle contient des composants d'une biomasse de thraustochytriales.

13. Composé anti-oxydatif et stabilisant d'acide gras selon la revendication 12, **caractérisée en ce qu'**elle contient des composants d'une biomasse d'ulkenia sp.

14. Composé anti-oxydatif et stabilisant d'acide gras selon au moins une des revendications précédentes, **caractérisée en ce qu'**on peut obtenir les composants de la biomasse au crypthecodinium sp. par un procédée de transestérification d'une biomasse différente du crypthecodinium sp.

15. Composé anti-oxydatif et stabilisant d'acide gras selon au moins une des revendications 11 à 14, **caractérisée en ce que**, par rapport au poids total du composé d'acide gras, il contient de 0,1 à 50,0 % de poids d'au moins un extrait selon au moins une des revendications 1 - 10, ainsi que 50,0 - 99,9 % de poids d'une biomasse différente du crypthecodinium sp.

16. Composé anti-oxydatif et stabilisant d'acide gras selon au moins une des revendications précédentes 11 - 15, **caractérisée en ce que**, par rapport à son poids total, il contient au moins 25,0 % de poids d'acide docosahexanoïque et / ou d'éther d'alkyle docosahexanoïque.

17. Procédé de fabrication d'un composé anti-oxydatif et stabilisant d'acide gras **caractérisé en ce qu'**au moins un extrait de crypthecodinium sp. ayant un effet anti-oxydatif est mélangé avec des composants d'une biomasse différente du crypthecodinium sp. la composition d'acide gras ne contenant aucun autre antioxydant.

18. Utilisation d'une composition à base d'acide gras selon au moins une des revendications 11 - 16 comme ingrédient actif ou composant dans un composé pharmaceutique.

19. Utilisation d'une composition à base d'acide gras selon au moins une des revendications 11 - 16 comme composant dans une préparation cosmétique.

20. Utilisation d'une composition à base d'acide gras selon au moins une des revendications 11 - 16 comme additif alimentaire et / ou ingrédient alimentaire.

21. Utilisation d'une composition à base d'acides gras selon au moins une des revendications 11-16 comme composant d'alimentation animale.
